# DEMANDE DE BREVET EUROPEEN

(11) **EP 4 066 866 A1**
(43) Date de publication de la demande: **05.10.2022**
(21) Numéro de dépôt: 21305392.9
(22) Date de dépôt: 29.03.2021
(51) Int. Cl.: A61L 15/32, A61L 27/36

(54) **GELEE DE WHARTON DECELLULARISEE - PROCEDE D'OBTENTION, BIOMATERIAU, COMPOSITIONS ET PRODUITS ASSOCIES**

(71) Demandeur: Université De Reims Champagne-Ardenne, 51100 Reims (FR)
(72) Inventeur: KERDJOUDJ, Halima, 51100 REIMS (FR); DUBUS, Marie, 51100 REIMS (FR); GANGLOFF, Sophie, 51170 Faverolles et Coemy (FR)
(74) Mandataire: Ipside

(57) **Abrégé**

La présente invention concerne une gelée de Wharton décellularisée, laquelle moins de 50 ng d'ADN double brin/mg de tissu sec et,
- au moins 5 µg de glycosaminoglycanes sulfatés /mg de tissu sec, en particulier au moins 7 µg de glycosaminoglycanes sulfatés /mg de tissu sec et plus particulièrement au moins 10 µg de glycosaminoglycanes sulfatés /mg de tissu sec et/ou
- au moins 8 µg de glycosaminoglycanes non sulfatés /mg de tissu sec, en particulier au moins 14 µg de glycosaminoglycanes non sulfatés /mg de tissu sec.

La présente invention concerne également un procédé de décellularisation d'un tissu conjonctif œdémateux, en particulier la gelée de Wharton qui permet d'obtenir la gelée de Wharton décellularisée précitée ainsi qu'un biomatériau et des produits et compositions contenant ladite gelée

## Description

### [Domaine technique]

La présente invention concerne une gelée de Wharton décellularisée, un procédé de décellularisation d'un tissu conjonctif permettant d'obtenir cette gelée et les différentes utilisations de ladite gelée.

Le développement de matériaux bioactifs de nouvelle génération favorisant la cicatrisation tissulaire est un défi pour la médecine réparatrice/régénératrice.

### [Art antérieur]

Dans les tissus, la matrice extracellulaire, de par sa capacité à protéger et à libérer les facteurs de croissance, joue un rôle actif dans les processus de réparation tissulaire. Les tissus périnataux tels que le placenta ou encore le cordon ombilical sont connus pour renfermer des quantités importantes de facteurs de croissance. De par sa structure fine, le placenta est fréquemment utilisé en dermatologie pour le traitement des grands brûlés ou encore en ophtalmologie pour la reconstruction de la cornée.

La gelée de Wharton, contenue dans le cordon ombilical a aussi été étudiée. Ainsi, le document FR 3 070 262 A1 décrit une composition contenant un broyat de gelée de Wharton viro inactivée. Cette gelée broyée et viro inactivée est obtenue par congélation à -80°C puis décongélation de morceaux de gelée afin de lyser les cellules que contient cette dernière. La gelée est ensuite déposée dans un bain d'eau pendant 3 heures afin de lyser les cellules restantes par pression osmotique. Elle est ensuite traitée dans un bain d'éthanol pendant une heure puis dans un bain de peroxyde d'oxygène. On réalise ensuite la neutralisation dans un bain de soude à un pH d'environ 8,5. La gelée est ensuite rincée avec du PBS puis mise dans deux bains successifs d'eau purifiée. La gelée est ensuite broyée. Ce procédé a le mérite de pouvoir être mis en œuvre à température ambiante mais il est long et il ne garantit pas une décellularisation suffisante. En particulier, il ne garantit pas une faible teneur en ADN double brin, ni la présence de facteurs de croissance dans la gelée traitée, ni l'intégrité mécanique de la matrice extracellulaire obtenue.

La publication intitulée « an overview of tissue and whole organ decellularization processes » de Peter Crapo, publiée en avril 211 dans la revue Biomaterials (32(12): 3233-3243. doi:10.1016/j.biomaterials.2011.01.057) décrit les diverses méthodes de décellularisation des tissus, lesquelles permettent d'obtenir la matrice extracellulaire de chaque tissu. Ces méthodes dépendent du type de tissu traité, de sa densité, de son épaisseur. Pour que la matrice extracellulaire puisse être utilisée comme biomatériau, son intégrité chimique et physique doit être conservée au maximum ; afin de ne pas avoir de réaction immune de rejet du sujet sur lequel on va implanter le biomatériau, il est nécessaire que la matrice extracellulaire traitée contienne moins de 50 ng d'ADN double brin par mg de matrice sèche ; il faut également vérifier l'absence de matériel nucléaire dans une section de la matrice traitée par coloration au 4',6-diamidino-2-phenylindole (DAPI) ou par coloration à l'hématoxyline et à l'éosine.

La publication intitulée « Decellularized Wharton's jelly for human umbilical cord as a novel 3D scaffolding material for tissue engineering applications » de Sushma Jadalannagari publiée dans la revue PLOS One, le 21 février 2017 décrit une matrice extracellulaire obtenue par décellularisation de gelée de Wharton. Le procédé utilisé est le suivant : le cordon ombilical récupéré en salle d'accouchement est transporté dans une solution de lactate de Ringer additionnée de pénicilline (800 U/mL), de streptomycine (9,1 mg/mL) et d'amphotéricine (0,25 mg/mL) à une température de 4°C. Le traitement de décellularisation est mis en œuvre 72 heures plus tard. Le procédé de décellularisation comprend le retrait des structures vasculaires et des membranes entourant la gelée, laquelle est découpée en larges pièces ovales. Ces pièces sont soumises à deux cycles de chocs osmotiques avec alternativement une solution hypertonique contenant du chlorure de sodium, du mannitol, du chlorure de magnésium et du chlorure de potassium avec une osmolarité approximative de 1 275 mOsm/L et une solution hypotonique de Triton X-100 (0,005%) dans de l'eau distillée et centrifugée à 5 000 tours/minutes à 4°C. Après ces deux cycles de chocs osmotiques, les tissus sont traités avec un détergent anionique (lauryl de sodium) et du succinate de sodium puis à une enzyme recombinante ciblant les acides nucléiques dans un tampon d'HCI dans de l'eau pendant 16 heures. On réalise ensuite une extraction avec un solvent contenant 40% d'éthanol pendant 10 minutes, sous agitation à 5 000 tours/min à 4°C. Le passage sur une résine échangeuse d'ions pendant 30 heures à température ambiante permet de retirer tous résidus de réactifs ou autres. La matrice décellularisée est lyophilisée avec 10% d'albumine humaine recombinante et 10% de DMSO dans un milieu standard avec une vitesse de refroidissement de -1°C/minute jusqu'à -180°C. La matrice extracellulaire ainsi obtenue est non cytotoxique et apte à être colonisée par des cellules mais elle a beaucoup perdu de sa teneur en glycosaminoglycanes, lesquels sont particulièrement abondants dans la gelée de Wharton.

Les glycosaminoglycanes (GAGs) sont des macromolécules glucidiques ayant une forte capacité de rétention d'eau. Ils jouent un rôle à la fois dans les propriétés mécaniques et chimiques de la matrice extracellulaire. Ils sont également importants dans la rétention et la protection des facteurs de croissance, lesquels vont ainsi pouvoir s'accumuler autour des cellules là où se trouvent les récepteurs cellulaires adéquats.

Ces facteurs peuvent être libérés par action protéolytique des cellules

Par ailleurs, la publication précitée indique que la matrice extracellulaire contient un seul facteur de croissance, le TGF beta. La baisse de la teneur en GAGs de la matrice extracellulaire obtenue engendre également une diminution de la quantité de facteurs de croissance présents dans cette dernière.

S'agissant du procédé de décellularisation, la publication précitée décrit un procédé agressif, long et qui doit être mis en œuvre à 4°C. L'agressivité du traitement engendre probablement la détérioration des GAGs qui sont lessivés avec les autres résidus de réaction, lors des différents rinçages.

### [Problèmes techniques à résoudre]

Un but de la présente invention est de proposer une gelée de Wharton décellularisée qui contient au moins 80% et en particulier au moins 90%, voire au moins 95% des GAGs contenus dans la gelée de Wharton native et plus particulièrement de la chondroïtine sulfate et/ou de l'acide hyaluronique contenus dans la gelée de Wharton ou dans la gelée de Wharton native.

Un autre but de la présente invention est de proposer une gelée de Wharton décellularisée qui présente des propriétés mécaniques proches de celles de la gelée de Wharton ou de la gelée de Wharton native et qui peut donc être utilisée en tant que biomatériau pour le comblement d'un défaut tissulaire par exemple, ou encore comme membrane/pansement pour la régénération/cicatrisation d'un tissu.

Un autre but de la présente invention est de proposer une gelée de Wharton décellularisée qui soit capable de libérer des facteurs de croissance.

Un autre but de la présente invention est de proposer une gelée de Wharton décellularisée qui présente des propriétés antimicrobiennes.

Un autre but de la présente invention est de proposer une gelée de Wharton décellularisée qui n'engendre pas de réponse inflammatoire exacerbée de la part de l'organisme avec lequel elle est mise en contact, notamment du fait de ses propriétés anti-inflammatoires.

En particulier, un but de la présente invention est de proposer une gelée de Wharton décellularisée qui n'engendre pas, en présence d'un stimulus inflammatoire, l'augmentation de la sécrétion des cytokines pro-inflammatoires par les monocytes et/ou qui ne modifie pas les propriétés phagocytaires des PNNs et/ou qui n'augmente pas la quantité de ROS et/ou qui n'augmente pas la sécrétion d'IL-8 des PNNs et/ou qui n'augmente pas la migration des fibroblastes.

Un autre but est de proposer une composition pharmaceutique utilisable par exemple, pour améliorer la cicatrisation ou traiter l'inflammation d'un tissu, notamment la peau ou la surface du globe oculaire.

Un autre but de la présente invention est de proposer un biomatériau pouvant servir de greffe ou d'implant afin de combler un défaut tissulaire ou d'augmenter une masse tissulaire.

Un autre but de la présente invention est de proposer un procédé de décellularisation d'un tissu conjonctif, en particulier un tissu conjonctif œdémateux et plus particulièrement la gelée de Wharton qui permet de n'altérer ni la composition chimique ni les propriétés mécaniques de cette dernière.

### [Brève description de l'invention]

La présente invention concerne une gelée de Wharton décellularisée, laquelle contient moins de 50 ng d'ADN double brin/mg de tissu sec et,
- au moins 5 µg de glycosaminoglycanes sulfatés /mg de tissu sec, en particulier au moins 7 µg de glycosaminoglycanes sulfatés /mg de tissu sec et plus particulièrement au moins 10 µg de glycosaminoglycanes sulfatés /mg de tissu sec et/ou
- au moins 8 µg de glycosaminoglycanes non sulfatés /mg de tissu sec, en particulier au moins 14 µg de glycosaminoglycanes non sulfatés /mg de tissu sec.

La gelée de l'invention peut contenir, en particulier moins de 40ng d'ADN /mg de tissu sec, plus particulièrement moins de 30 ng d'ADN/mg de tissu sec. Elle peut également contenir moins de 20 ng d'ADN/mg de tissu sec, plus particulièrement moins de 10ng d'ADN/mg de tissu sec et encore plus particulièrement moins de 8 ng d'ADN/mg de tissu sec.

Plus particulièrement, la présente invention concerne une gelée de Wharton telle que précitée qui contient :
- au moins 5 µg chondroïtine sulfate /mg de tissu sec, en particulier au moins 7 µg de chondroïtine sulfate /mg de tissu sec et plus particulièrement au moins 10 µg de chondroïtine sulfate /mg de tissu sec et/ou
- au moins 8 µg d'acide hyaluronique /mg de tissu sec, en particulier au moins 14 µg d'acide hyaluronique /mg de tissu sec.

Les Inventeurs ont en effet constaté qu'une telle gelée de Wharton décellularisée notamment de sa faible teneur en ADN double brin pouvait être bien tolérée par l'organisme d'un sujet, notamment un mammifère et plus particulièrement un humain. La teneur en GAGs et plus particulièrement en chondroïtine sulfate et/ou en acide hyaluronique de cette gelée décellularisée, lui confère les mêmes propriétés mécaniques et physicochimiques que la gelée de Wharton non décellularisée, en particulier, en ce qui concerne l'hygroscopie et la capacité à libérer des facteurs de croissance.

La gelée de l'invention contenant les mêmes constituants et dans les mêmes proportions que la gelée de Wharton non traitée, elle est apte à être utilisée comme médicament ou comme ingrédient actif dans une composition cosmétique. Elle peut également, du fait de la présence des facteurs de croissance, être utilisée pour améliorer la cicatrisation.

### [Description détaillée de l'invention]

Avantageusement, la gelée de Wharton de l'invention peut présenter une porosité supérieure à 60%, en particulier supérieure ou égale à 70% et plus particulièrement égale ou supérieure à 80% et inférieure ou égale à 85%. Une porosité égale à 70% correspond sensiblement à la porosité de la gelée de Wharton native. Lorsque la gelée de l'invention est utilisée en tant que biomatériau destiné à être colonisé par des cellules du sujet avec lequel elle est mise en contact, sa porosité lui permet d'être facilement colonisée et de former ensuite un tissu similaire au tissu du sujet.

Quel que soit le mode de réalisation, la gelée de l'invention peut contenir, en outre, au moins 0,70 mg de collagène /mg de tissu sec et en particulier au moins 0,80 mg de collagène /mg de tissu sec. Cette proportion de collagène correspond sensiblement à celle du collagène dans la gelée de Wharton native ; le collagène confère une bonne élasticité à la gelée de l'invention ce qui est un avantage lorsqu'elle est utilisée pour une greffe de tissu, par exemple ou comme implant tissulaire. La gelée de Wharton de l'invention présente ainsi les mêmes propriétés mécaniques que la gelée native.

La gelée de l'invention présente des pores de taille plus élevée que celle des pores de la gelée de Wharton native. Ceci est probablement dû au traitement de décellularisation, en particulier à l'étape de congélation lorsqu'elle est présente. Néanmoins pour un usage en tant que biomatériau, la taille des pores et la porosité globale de la gelée de Wharton selon l'invention ne sont pas gênantes.

La gelée de Wharton selon l'invention peut être hydratée ou se présenter sous sa forme sèche, c'est-à-dire lyophilisée. La forme humide (hydratée) est avantageusement obtenue par réhydratation de la gelée lyophilisée. La lyophilisation permet de conserver la gelée à température ambiante sans dénaturation. Le produit obtenu est facile à transporter, stocker et utiliser.

A titre d'exemple, la gelée de Wharton de l'invention peut présenter un module d'élasticité linéaire d'au moins 12 MPa. C'est en particulier le cas de la gelée de l'invention sous une forme lyophilisée.

La gelée de Wharton selon l'invention peut se présenter sous la forme d'un morceau de taille et de forme non limitées ; elle peut se présenter sous une forme divisée, c'est-à-dire sous la forme d'une pluralité de morceaux de taille et de forme non limitées et en particulier sous la forme d'une poudre de granulométrie non limitée ou de granulés. La gelée de Wharton de l'invention est obtenue à partir du cordon ombilical, celui-ci étant avantageusement un humain. La gelée de Wharton de l'invention peut donc provenir d'un cordon ombilical humain.

Du fait de la teneur élevée en GAGs de la gelée de l'invention, laquelle est quasi identique à celle de la gelée de Wharton native, les Inventeurs ont constaté qu'elle était susceptible de libérer/contenir au moins un peptide antimicrobien et/ou au moins un facteur de croissance et/ou au moins un peptide favorisant la cicatrisation tissulaire. Avantageusement, elle contient au moins un peptide antimicrobien et au moins un peptide favorisant la cicatrisation tissulaire et au moins un facteur de croissance. Selon un mode de réalisation particulier, le peptide anti-microbien est choisi parmi le fibrinogène chaîne beta (en Anglais « Fibrinogen beta chain »), le fibrinogène chaine alpha (« Fibrinogen alpha chain ») et la cytokératine-10 (« Keratin, type I cytoskeletal 10 »), ledit facteur de croissance est choisi parmi VEGF, HGF et TGF-beta et ledit peptide favorisant la cicatrisation est choisi parmi la ténascine (« Tenascin »), la cytokératine-6A (« Keratin, type II cytoskeletal 6A »), le collagène alpha-1 (III) (« Collagen alpha-1 (III) chain »), la fibronectine (« Fibronectin »), la cytokératine -1 (« Keratin, type II cytoskeletal 1 »), la cytokératine-17 (« type I cytoskeletal 17) et la cytokératine-9 (« Keratin, type I cytoskeletal 9 »).

Avantageusement, la gelée de Wharton contient et donc libère les trois peptides antimicrobiens précités, les 7 peptides favorisant la cicatrisation tissulaire précités, du VEGF, du TGF-beta et du HGF. La gelée de Wharton décellularisée de l'invention a donc des propriétés antimicrobiennes et régénératrices.

Le VEGF (facteur de croissance de l'endothélium vasculaire) présent dans la gelée de l'invention va permettre la formation de vaisseaux sanguins et la vascularisation tissulaire. Lorsque la gelée de l'invention est utilisée en tant que biomatériau pour le traitement d'un défaut tissulaire ou d'une altération tissulaire ou dans une composition pharmaceutique, la présence du VEGF permet aux cellules du sujet qui auront colonisé le biomatériau de l'invention ou qui sont en contact avec la gelée de l'invention de produire des vaisseaux sanguins ; le biomatériau de l'invention permet ainsi de remplacer le tissu manquant par un tissu comportant les cellules du sujet et qui est de plus vascularisé, de manière quasi naturelle. La gelée utilisée dans le traitement d'une lésion tissulaire favorise également la revascularisation de cette dernière par simple contact.

Le TGF-beta (incluant au sens de l'invention les trois isoformes présentes chez les mammifères et notamment chez l'humain) régule le processus inflammatoire, notamment la quantité de lymphocytes T et leur différenciation. Il est également connu pour la prolifération des lymphocytes B.

Le HGF (facteur de croissance des hépatocytes) qui est présent dans la gelée de Wharton (il est secrété par les cellules mésenchymateuses) reste donc présent dans la gelée de l'invention. Il est connu pour son rôle dans le traitement des blessures et la régénération des organes chez l'adulte.

Les inventeurs ont également constaté que la gelée de l'invention possède des propriétés antibactériennes qui ne sont pas forcément dues à la présence des peptides antibactériens précités. Il a été constaté que la gelée de l'invention présente des propriétés bactériostatiques, notamment sur des bactéries Gram+, comme *Staphylococcus aureus* et *Staphylococcus epidermidis* et sur des bactéries Gram-, notamment *Pseudomonas aeruginosa* et *Escherichia coli.* La gelée de l'invention semble pouvoir tuer les bactéries par contact ce qui est un grand avantage pour l'utilisation de la gelée de l'invention en tant qu'agent antibactérien tout comme biomatériau.

La gelée de l'invention permettrait également la différenciation des macrophages en macrophages de type M2 ayant un profil pro-cicatrisant.

Les inventeurs ont également constaté que de manière surprenante, la gelée de Wharton de l'invention est exempte de propriétés immunogènes et/ou présente des propriétés anti fibrotiques. En particulier, elle n'induit pas de réaction inflammatoire exacerbée en présence de monocytes circulants dont elle n'augmente pas la sécrétion de cytokines pro-inflammatoires. Les Inventeurs ont en effet constaté que lorsque la gelée de Wharton de l'invention est mise en contact avec des monocytes humains circulants, la sécrétion de cytokines pro-inflammatoires de ces derniers est inférieure à la sécrétion desdits monocytes mis au contact d'un agent inflammatoire de référence. L'agent inflammatoire est, par exemple, un lipopolysaccharide (LPS).

De plus, les Inventeurs ont constaté que la gelée de l'invention ne bloque pas la phagocytose des polynucléaires neutrophiles. Ils ont également constaté que la gelée de l'invention n'augmentait pas la production d'espèces réactives à l'oxygène (ROS) lesquelles sont impliquées dans le mécanisme d'exacerbation de la réponse inflammatoire. De même, la sécrétion d'IL-8 par les polynucléaires neutrophiles n'est pas augmentée par la gelée de l'invention. La gelée de l'invention ne génère donc aucune réaction du système immunitaire du sujet de part le fait de sa faible teneur en ADN double brin, laquelle confirme l'absence de cellules dans la gelée de l'invention et de par sa composition chimique riche en GAGs.

Par ailleurs, les Inventeurs ont constaté que la gelée de Wharton décellularisée de l'invention n'induit pas de migration des fibroblastes ce qui suggère que la gelée de l'invention ne va pas conduire à une hyper cicatrisation,(caractère antifibrotique de la gelée de l'invention) laquelle altèrerait le tissu ou l'organe en contact avec la gelée de l'invention.

Ainsi, la gelée selon l'invention se caractérise par le fait que l'augmentation de la sécrétion des cytokines pro inflammatoires par les monocytes circulants en présence de ladite gelée est inférieure à celle due à un facteur inflammatoire, en particulier un lipopolysaccharide (LPS) et/ou en ce qu'en présence de ladite gelée le nombre de cellules phagocytées par les PNN est égal au moins 70% et en particulier 80% ou plus du nombre de cellules phagocytées en l'absence de ladite gelée et/ou en ce que la quantité de ROS secrétés par les PNNs en présence de ladite gelée est inférieure ou égale à la quantité de ROS secrétés par les PNNs en présence d'un stimulus inflammatoire, en particulier un lipopolysaccharide et/ou en ce que le ratio [production d'IL-8 par les PNN en présence d'un stimulus inflammatoire, en particulier un polysaccharide LPS en présence de ladite gelée]/[la production d'IL-8 par les PNNs en présence de cellules basales] est inférieur ou égal à 1,5 et/ou en ce que le pourcentage de fibroblastes ayant migrés pendant 48h en présence de ladite gelée est inférieur au pourcentage de fibroblastes ayant migrés pendant 48h en l'absence de ladite gelée.

La présente invention concerne également un biomatériau comportant ou constitué de la gelée de Wharton de l'invention. Ce biomatériau peut se présenter sous une forme solide, comme des morceaux de gelé de l'invention ; il peut se présenter sous la forme de particules, d'une poudre, d'une membrane ou d'une mousse, par exemple. Il peut également se présenter sous une forme liquide plus ou moins visqueuse ou sous la forme d'un gel. La gelée de l'invention peut être sous sa forme hydratée ou sèche (lyophilisée). Elle peut provenir également de la réhydratation de la forme lyophilisée de la gelée. Le biomatériau de l'invention peut être hydraté avant son utilisation ou être mis au contact avec l'organisme du sujet à l'état lyophilisé, le contact avec les fluides corporels, notamment le liquide interstitiel réhydratant le biomatériau.

La présente invention concerne également une composition pharmaceutique ou cosmétique qui, de manière caractéristique, comprend en tant qu'ingrédient actif, la gelée de Wharton de l'invention.

Du fait de ses propriétés cicatrisantes et régénératrices, la composition de l'invention peut être utilisée à fins cosmétiques pour l'hydratation de la peau ou la diminution des rides (du fait de la richesse en acide hyaluronique de la gelée de l'invention), par exemple.

La présente invention concerne la gelée de l'invention ou la composition pharmaceutique de l'invention, pour son utilisation en tant que médicament en particulier pour son utilisation en tant qu'agent favorisant la cicatrisation, agent réduisant l'inflammation et/ou agent hydratant pour le traitement d'une maladie ou dans une condition d'écart à la norme non pathologique nécessitant un tel traitement. La gelée de l'invention peut être utilisée pour réparer les tissus lésés mais aussi pour les régénérer.

La composition pharmaceutique ou la composition cosmétique peuvent être sous la forme d'une pommade, d'une lotion, d'une solution, d'un collyre ou d'une émulsion, par exemple.

La gelée peut se trouver sous sa forme lyophilisée ou sous sa forme réhydratée dans les compositions précitées. Elle peut être réhydratée avant son utilisation ou être utilisée sèche et s'hydrater au contact d'un fluide corporel du sujet, par exemple les larmes, la lymphe ou le liquide interstitiel.

La composition pharmaceutique de l'invention ou la gelée de l'invention peut être utilisée pour le traitement d'une lésion tissulaire de divers organes ou tissus comme, par exemple, la peau, le tissu épithélial de l'œil, un tissu conjonctif comme les fascias, les tissus adipeux, le tissu musculaire voire le tissu nerveux.

La présente invention concerne également un produit choisi parmi un implant, un pansement, un greffon, une compresse, une bio encre, un agent d'enrobage cellulaire qui contient ou est constitué de la gelée selon l'invention.

La présente invention concerne également un procédé de décellularisation d'un tissu conjonctif œdémateux, en particulier la gelée de Wharton qui permet l'obtention de la gelée de l'invention. Selon ce procédé, de manière caractéristique,
- on traite ledit tissu de préférence préalablement congelé à -20°C avec une solution de tensioactif anionique en maintenant la température à 37°C ;
- après rinçage à l'eau distillée à température ambiante, on ajoute une DNase et l'on maintient à 37°C ;
- on rince ensuite avec du PBS et éventuellement on lyophilise le tissu traité.

Les durées de traitement pour chaque étape sont à ajuster en fonction de la taille des morceaux traités.

Le tissu peut être réduit en morceaux plus fins, par broyage, par exemple, avant le procédé de décellularisation de l'invention, après ou après la lyophilisation.

Ce procédé assure une décellularisation rapide et adaptée pour un usage de la gelée de Wharton en tant que biomatériau sans dégradation physicochimique de sa matrice extracellulaire.

Le tensioactif anionique peut être choisi parmi les octylphénoxypolyéthoxyéthanol soluble en milieu aqueux et contenant en moyenne 10 moles d'oxyde d'éthylène (Triton X-100), les éthoxylates d'alcool secondaire (Tergitol^{®} 15-S-7), le tris dodécyl sulfate, le cholate de sodium extrait de la bile bovine, le dodécyl sulfate de sodium, l'hydrate de N-lauroylsarcosinate de sodium, le déoxycholate de sodium et le dodécyl sulfate de sodium, par exemple.

Avantageusement, le tensioactif est mis au contact de la gelée de Wharton pendant 30 min à 90 min et notamment pendant 60 min.

Avantageusement la DNase est laissée au contact de la gelée de Wharton pendant au moins 12 heures et en particulier pendant 24 heures.

De préférence, le tissu est congelé à -20°C avant d'être traité par le tensioactif anionique. Cette congélation permet de fragiliser la membrane des cellules facilitant leur dissolution par le tensioactif anionique. La veine et les artères peuvent être retirées avant ou après la congélation à -20°C.

Le procédé de l'invention peut s'appliquer en particulier sur des morceaux de 1cmX1 cmX1 mm. Les morceaux congelés sont avantageusement rincés sous agitation avec de l'eau distillée jusqu'à décongélation.

La température de 37°C permet de ne pas altérer la gelée de Wharton et d'activer l'action de la DNase et du tensioactif, lequel lyse la membrane cellulaire. Le procédé de l'invention est ainsi beaucoup plus rapide et aisé à mettre en œuvre que ceux de l'art antérieur.

Avantageusement, on lyophilise la gelée décellularisée. Cette lyophilisation a lieu en trois étapes, une étape de congélation à -20°C pendant environ 24 heures puis une congélation à -80°C pendant au moins 24 heures et de préférence 48 heures et une dessiccation sous-vide à -80°C pendant 24 heures ou plus qui a pour but de sublimer les molécules d'eau congelées.

### [Définitions]

Les termes « gelée de Wharton » désignent le tissu conjonctif gélatineux présent dans le cordon ombilical d'un mammifère dont la veine et les deux artères naturellement incluses dans le tissu conjonctif ont été retirées. Au sens de la présente invention le terme « gelée de Wharton » englobe la gelée de Wharton seule et la gelée de Wharton comprenant au moins une partie de la membrane amniotique qui la recouvre.

Le terme « gelée de Wharton native » désigne une gelée de Wharton au sens de l'invention, laquelle a été soumise aux mêmes étapes que le procédé de l'invention excepté la mise en contact avec le tensioactif anionique et la mise au contact avec la DNase.

Le terme « lyophilisation » désigne une technique visant à dessécher un produit préalablement surgelé par sublimation. Plus précisément, le liquide à ôter du produit est dans un premier temps transformé en glace par congélation, puis par une dessiccation primaire, sous vide, la glace est sublimée ; enfin par une dessiccation secondaire, les molécules d'eau à la surface du produit sont extraites par désorption. Le terme « implant » désigne tout objet tridimensionnel formé d'un biomatériau et apte, par exemple à remplacer une partie d'un tissu ou d'un tissu d'un organe. L'implant peut ainsi être inséré ou greffé au niveau du derme, de l'épiderme, sous le derme et/ou l'épiderme ; il peut s'agir d'un implant mammaire, par exemple, de reconstruction ou d'augmentation mammaire. Il peut également s'agir d'un implant dentaire.

Les termes « absence de propriétés immunogènes » signifie au sens de l'invention l'absence d'augmentation de la sécrétion de cytokines pro-inflammatoires de monocytes circulants mis au contact du tissu de l'invention et/ou l'absence de blocage de la phagocytose de polynucléaires neutrophiles et/ou l'absence d'augmentation de la quantité d'espèces réactives à l'oxygène libérée (ROS) et/ou l'absence d'augmentation de la quantité d'IL-8 produit par les polynucléaires neutrophiles en présence du tissu de l'invention.

Les termes « propriété anti-fibrotique » désignent l'absence de migration ou la faible migration (moins 10%) des fibroblastes en présence du tissu de l'invention.

Une lésion tissulaire désigne, au sens de l'invention, tout modification pathologique ou traumatologique d'un tissu, induite par une plaie, une blessure, coupure ou conséquence d'une réaction infectieuse et/ou inflammatoire ou brûlure ou ulcère de tout dommage tissulaire. Le tissu biologique peut être, par exemple, le tissu conjonctif, le tissu musculaire, le tissu nerveux ou le tissu épithélial. Ce tissu peut être localisé sur divers organes du corps du sujet. Ainsi le tissu peut être un tissu conjonctif (fascias, tissu adipeux, veines, artères os, yeux, nerfs, muscles), en particulier un tissu conjonctif dense (derme, tendon, cornée, par exemple), un tissu musculaire ou un tissu nerveux.

S'agissant de la peau, une lésion cutanée désigne, au sens de l'invention, tout modification pathologique ou traumatologique de l'épiderme, du derme ou du derme et de l'épiderme induite par une plaie ou une blessure ou conséquence d'une réaction infectieuse et/ou inflammatoire ou brûlure ou ulcère de tout dommage tissulaire. Par exemple, l'eczéma, l'herpès, le zona une brûlure thermique ou chimique un ulcère, une coupure, une cicatrice, une cicatrisation retardée ou non jointe sont des lésions cutanées au sens de l'invention.

Le terme « bio impression » désigne tout procédé d'ingénierie tissulaire utilisant les principes de l'impression 3D appliqués à la matière vivante cellulaire ou à des biomatériaux et procédant à l'assemblage couche par couche des constituants d'un tissu biologique selon des architectures prédéfinies par conception numérique. La bio impression permet de produire des greffons pour la médecine régénératrice ou des modèles physiologiques à des buts de recherche ; elle permet ainsi de remplacer les tests pharmacologiques sur les animaux.

Le terme « facteur d'enrobage et d'aide à la bio impression » désigne une matrice dans laquelle les biomatériaux ou cellules destinées à la bio impression sont compris. Cette matrice enrobe les biomatériaux ou cellules lors de la bio impression, elle sert de support et de protection à ces derniers. L'ensemble constitué de la matrice et des biomatériaux ou cellules destinées à la bio impression est désigné par le terme « bio encre ».

Le terme « biomatériau » désigne au sens de l'invention tout matériau apte à interagir avec les systèmes biologiques, qu'il participe à la constitution d'un dispositif à visée diagnostique ou à celle d'un substitut de tissu ou d'organe ou encore à celle d'un dispositif de suppléance (ou d'assistance) fonctionnelle. Un biomatériau peut ainsi être considéré comme tout matériau utilisé pour remplacer une partie ou une fonction du corps de manière sure et fiable, acceptable d'un point de vue physiologique.

Les termes « température ambiante » désignent une température supérieure ou égale à 20°C et inférieure ou égale à 25°C.

Le terme « greffon » désigne un fragment de tissu biologique apte à être greffé.

Les termes « tissu sec » désignent un tissu contenant 20% d'eau ou moins, notamment 10% d'eau et pouvant être obtenu par congélation à -20°C pendant 24h puis congélation jusqu'à -80°C et maintien de la température à -80°C pendant 24 heures ou 48 heures puis mise sous vide à -80°C pendant 24 heures.

### [Figures]

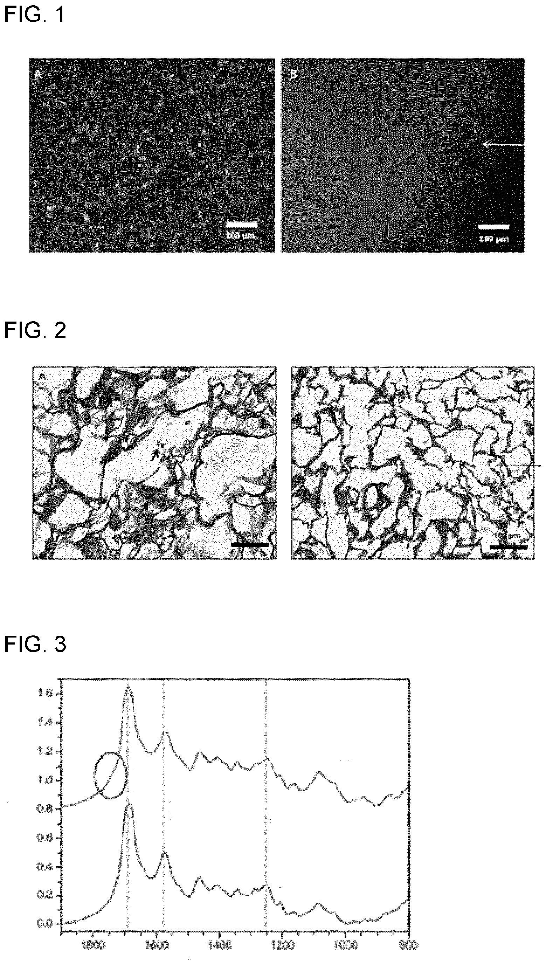

La Fig. 1 représente une image au microscope à fluorescence des noyaux après marquage au DAPI (intercalant d'ADN), dans la gelée native (A) et dans le tissu décellularisé de l'invention (B) (grossissement x10, barres d'échelles : 100 µm).
La Fig. 2 représente des photographies de vues de coupes histologiques colorées à l'hématoxyline/éosine/safran de la gelée native (A) et du tissu décellularisé de l'invention (B) permettant la visualisation du collagène et les noyaux cellulaires (Barres d'échelles : 100 µm, épaisseur des coupes : 5 µm) ; les flèches indiquent les noyaux dans la gelée native et la flèche montre l'absence de noyaux dans la gelée de l'invention.
La Fig. 3 représente les spectres d'intensité vs longueur d'onde (en cm⁻¹) obtenus par micro-spectroscopie infrarouge (tissu natif : courbe du haut, tissu décellularisé : courbe en bas), l'épaulement lié au acides nucléiques est entouré.

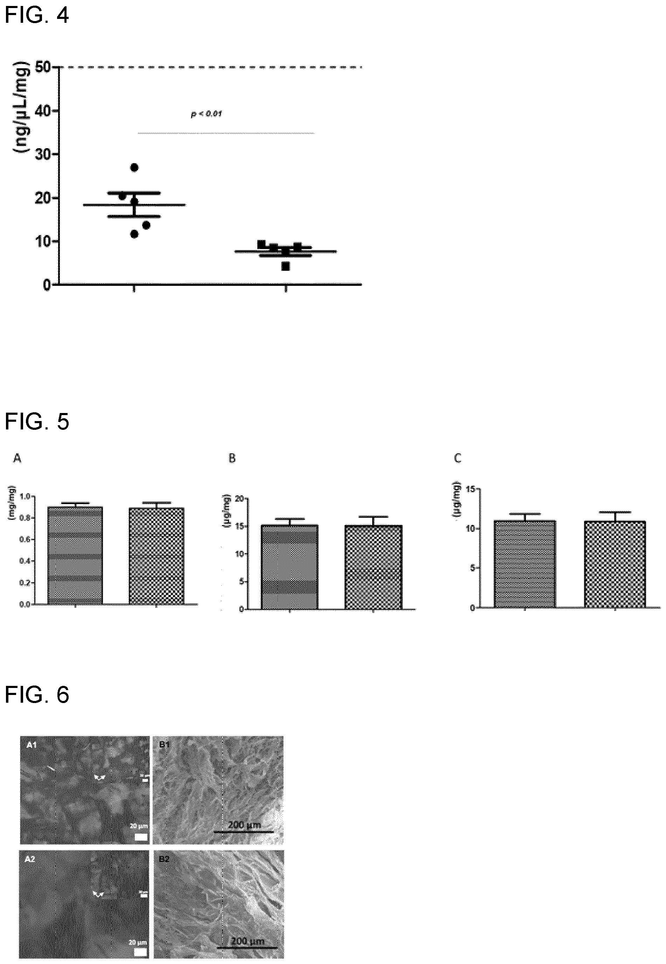

La Fig. 4 représente la quantité d'ADN (ng/µL/mg de tissus sec ) dans de la gelée native (à gauche) et dans le tissu décellularisé de l'invention (à droite) (Test de Mann Whitney, (n=6)).
La Fig. 5 représente la quantité de collagène (mg/mg de tissu sec) (A), d'acide hyaluronique (µg/mg de tissu sec) (B) et de chondroïtine sulfate (µg/mg de tissu sec) (C) dans le tissu natif (colonnes de gauche) et dans le tissu décellularisé de l'invention (colonnes de droite).

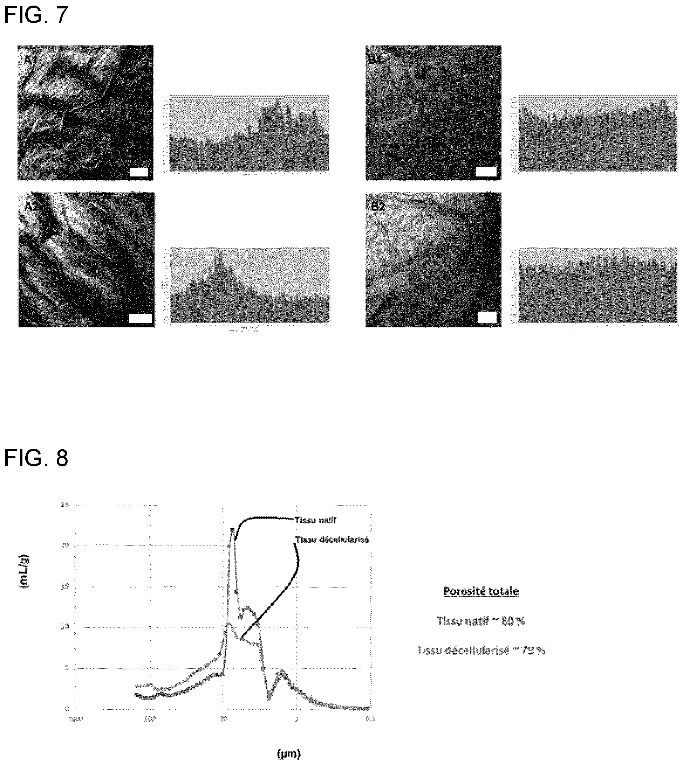

La Fig. 6 représente des vues au microscope optique à lumière polarisée de tissus natifs (A1) et décellularisé selon l'invention (A2) et des vues au microscope électronique à balayage, B1 étant une vue du tissu natif et B2 du tissu de l'invention ( A barres d'échelles 20 µm et inserts : polarisation 45°; B : barres d'échelles 200 µm). La Fig. 7 représente des vues au microscope confocale à balayage laser par génération de la seconde harmonique en conditions sèches (à gauche) et hydratées (à droite) du tissu natif (en haut) et du tissu décellularisé de l'invention (en bas) (Barres d'échelles 50 µm) ainsi que des histogrammes obtenus avec l'outil « directionnality » de Image J, représentants la directions des fibres de collagène pour chaque image ; La Fig. 8 représente la quantité de mercure (mL/g) introduite dans un gramme de tissu en fonction du rayon d'accès des pores (en µm) pour le tissu natif (courbe haute) et le tissu décellularisé (courbe basse).

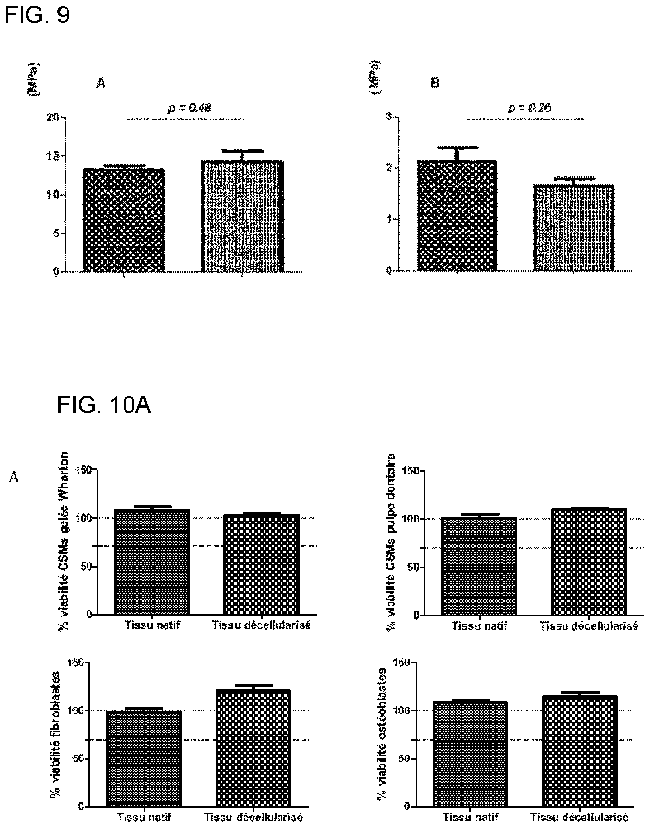

La Fig. 9 représente les modules d'élasticité obtenus par test de traction du tissu natif et du tissu décellularisé, en conditions sèches (A) et humides (B) (Test de Mann Whitney, (n=6)), pour chaque mesure la colonne de gauche représente le module d'élasticité du tissu natif et la colonne de droite représente le module d'élasticité pour le tissu de l'invention.

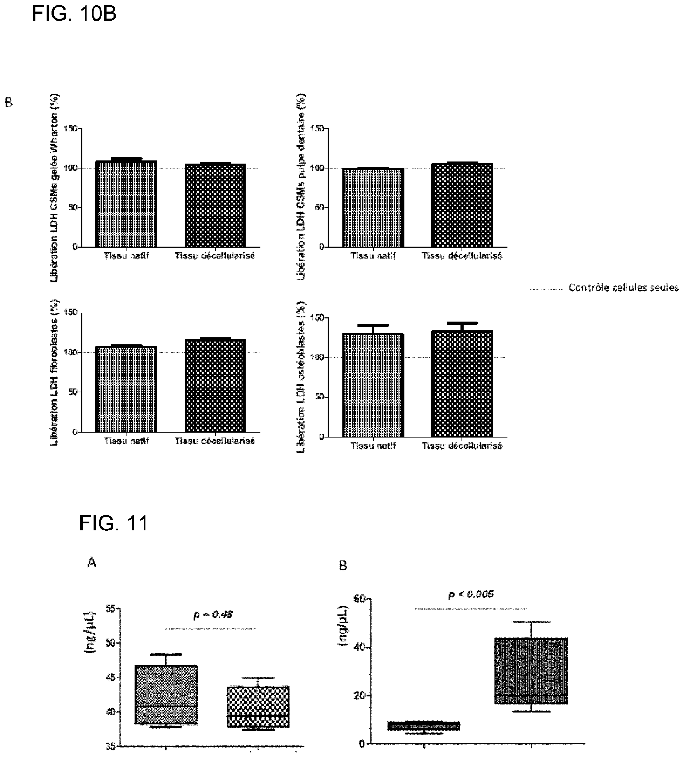

La Fig. 10A représente le pourcentage de viabilité des CSMs de la gelée de Wharton, des CSMs de la pulpe dentaire, des fibroblastes et des ostéoblastes dans le tissu natif et dans le tissu de l'invention, la ligne en pointillés du haut représente le contrôle (cellules seules) et la ligne en pointillés la plus basse représente le seuil des 70% ; La Fig. 10B représente le pourcentage de libération de la lactate déshydrogénase (LDH) des Cellules stromales (CSMs) de la gelée de Wharton, des CSMs de la pulpe dentaire et des fibroblastes et des ostéoblastes cultivé(e)s en monocouche et mises en présence du tissu natif ou du tissu décellularisé de l'invention pendant 24h (la ligne en pointillés du haut représente le contrôle (cellules seules);
La Fig. 11 représente la quantité de l'ADN (en ng/µL) de CSMs de la gelée de Wharton cultivées en présence des tissus natifs et décellularisés à 24h de culture (A) (tissu natif à gauche, tissu de l'invention à droite ) et la quantité de l'ADN de CSMs (en ng/µL) dans le tissu de l'invention (à gauche) et dans le tissu de l'invention après culture de CSMs en présence de ce dernier (à droite) à 7 jours de culture (B).

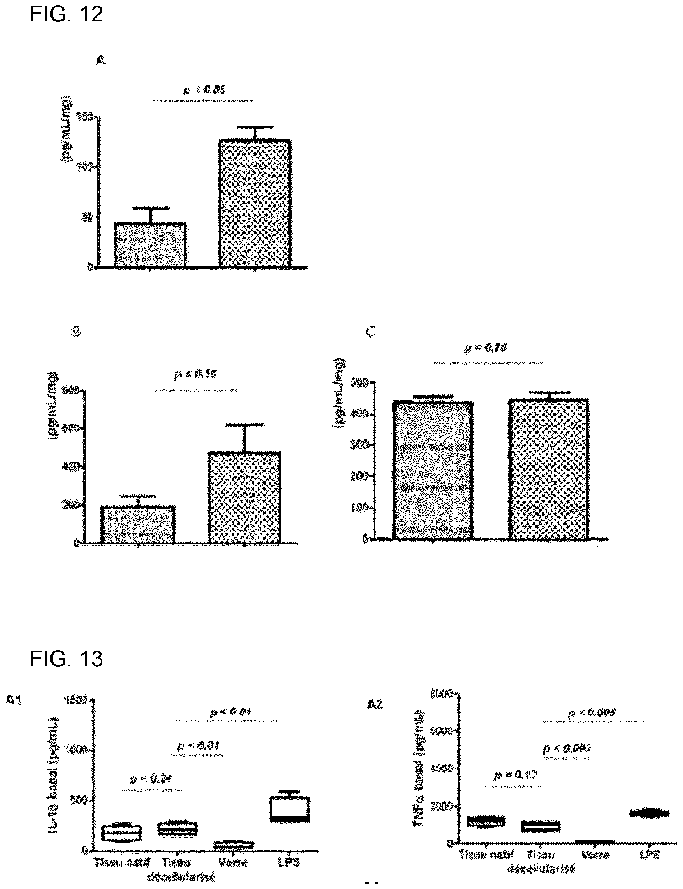

La Fig. 12 représente la concentration en facteurs de croissance (VEGF : 12A ; HGF : 12B ; TGF-beta : 12C) mesurée par ELISA dans du milieu biologique après 72h d'incubation à 37°C des tissus natifs (à gauche) et de l'invention (à droite). Test de Mann Whitney, (n=6).

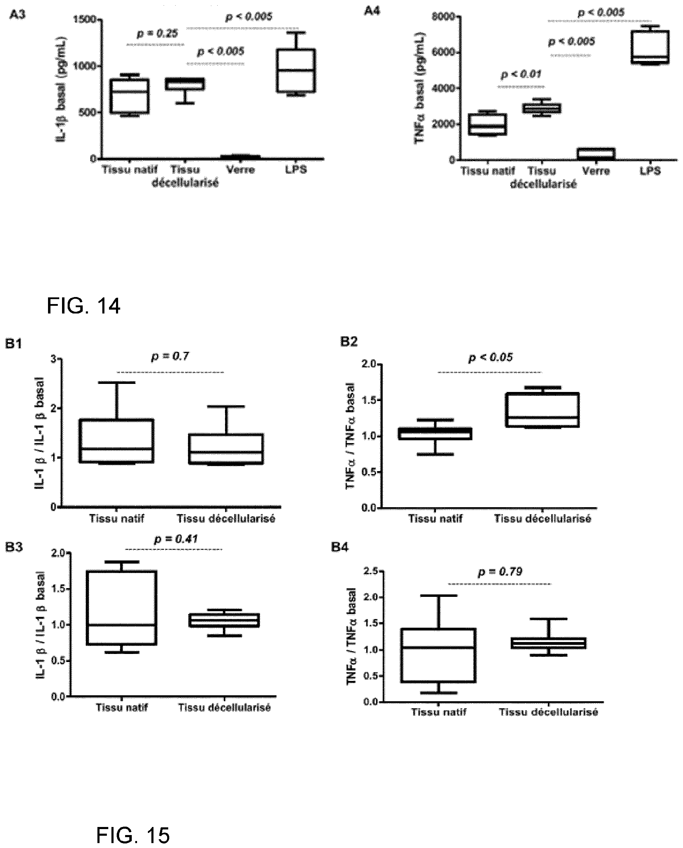

La Fig. 13 représente la concentration mesurée par ELISA de la sécrétion des cytokines pro-inflammatoires IL-1β (A1 et A3) et TNFα (A2 et A4) par des monocytes humains circulants mis en contact avec les tissus natifs et décellularisés selon l'invention pendant 4h (A1 et A2) et 24h (A3 et A4). (Verre : contrôle des monocytes en présence d'une lamelle de verre inerte et LPS : contrôle inflammatoire (10 ng/mL). Test de Mann Whitney, (n=6)) ;
La Fig. 14 représente les rapports entre la sécrétion d'IL-1β (B1 et B3) et de TNF-α (B2 et B4) en conditions basales par rapport à leur sécrétion en présence d'un stimulus inflammatoire (LPS, 10 ng/mL), par des monocytes humains en contact avec les tissus natifs et décellularisés selon l'invention, pendant 4h (B1 et B2) et 24h (B3 et B4). Test de Mann Whitney, (n=6).

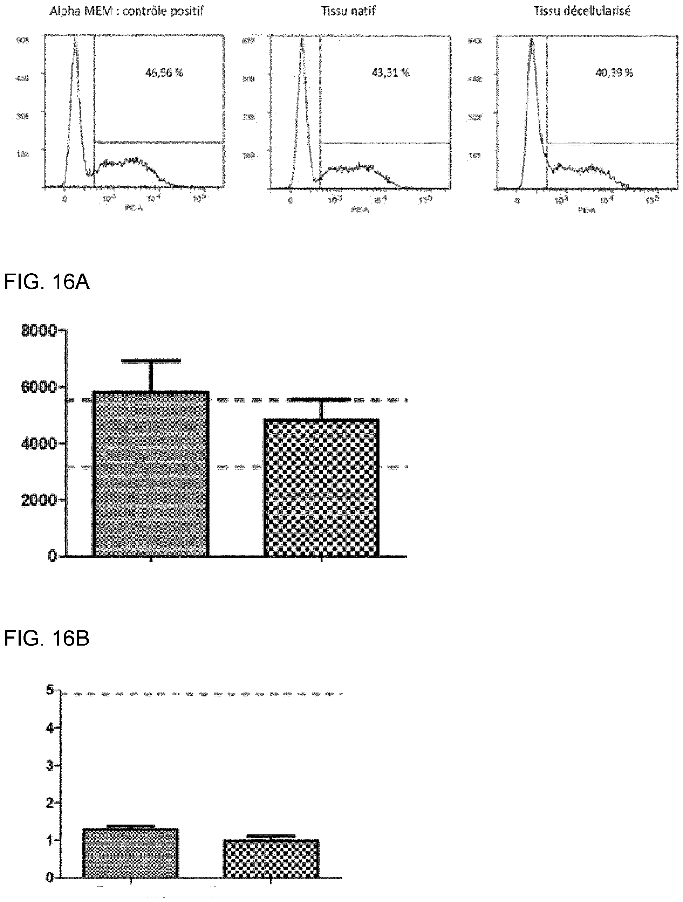

La Fig. 15 représente le nombre de cellules ayant phagocyté les particules fluorescentes mesuré par cytométrie de flux. Les polynucléaires neutrophiles (PNNs) humains mis en contact de particules pHRodo^{®} (mimant les bactéries) en présence pendant 1h de surnageants obtenus respectivement à partir du tissu natif et du tissu décellularisé de l'invention (surnageants obtenus après 72h d'incubation dans un milieu biologique à 37°C).
La Fig. 16A représente l'intensité médiane de la fluorescence qui correspond à la quantité de la production des ROS par les PNNs mesurée par cytométrie de flux, en présence des tissus natifs (à gauche) et du tissu décellularisés de l'invention (à droite), sur la Fig. 16, la ligne en pointillés haute représente l'intensité médiane obtenue avec le stimulus inflammatoire (LPS 10 ng/mL), la ligne en pointillés basse représente l'intensité médiane obtenue pour les cellules basales.
La Fig. 16B, représente les rapports entre la production intracellulaire des ROS par les PNN, mesurée par cytométrie de flux, en conditions basales par rapport à leur production en présence d'un stimulus inflammatoire (LPS, 10 ng/mL), par des PNN en contact avec les tissus natifs et décellularisés selon l'invention. La ligne en pointillés représente le ratio LPS/basal obtenu pour les cellules basales.

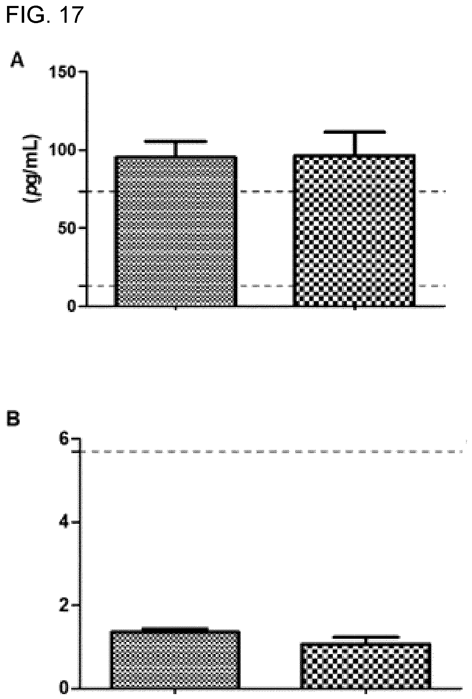

La Fig. 17A représente la concentration en IL-8 secrété par les PNN mesurée par ELISA, en présence des tissus natifs (à gauche) et du tissu décellularisés de l'invention (à droite), sur la Fig. 17A, la ligne en pointillés haute représente la concentration en IL-8 obtenue avec le stimulus inflammatoire (LPS 10 ng/mL), la ligne en pointillés basse représente la concentration en IL-8 obtenue pour les cellules basales,
La Fig. 17B représente les rapports entre la sécrétion d'IL-8 par les PNN, mesurée par ELISA, en conditions basales par rapport à la sécrétion en présence d'un stimulus inflammatoire (LPS, 10 ng/mL), par des PNN en contact avec les tissus natifs (colonne de gauche) et décellularisés selon l'invention (colonne de droite). La ligne en pointillés représente le ratio LPS/basal obtenu pour les cellules basales.

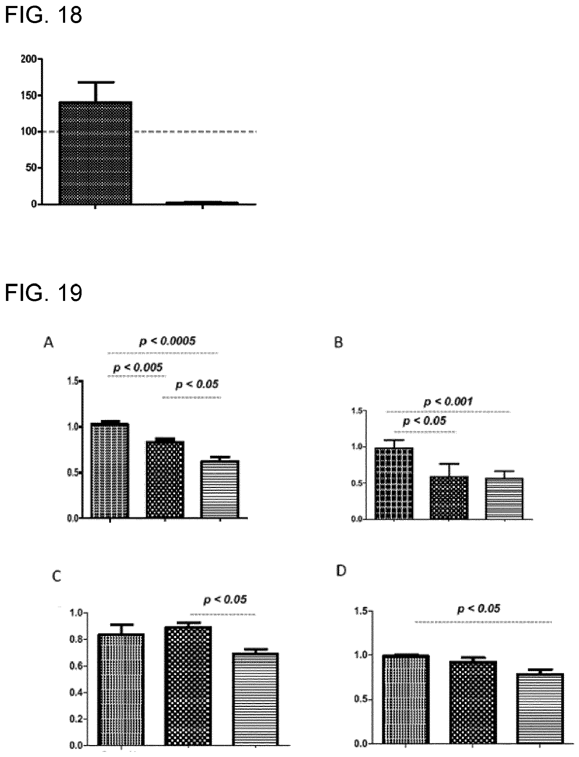

La Fig. 18 représente le pourcentage de fibroblastes ayant migrés pendant 48h en présence des tissus natifs (à gauche) et décellularisés selon l'invention (à droite). La ligne en pointillés représente le pourcentage de fibroblastes ayant migrés pendant 48h sans présence de tissu natif ou décellularisé.
La Fig. 19 représente la densité optique mesurée après 24h d'incubation de S. *aureus* (A), *S*. *epidermidis* (B), *E. coli* (C) et *P. aeruginosa* (D) en présence des tissus natifs (colonne à carreaux à gauche) et décellularisés de l'invention (colonne rayée horizontalement, la plus à droite) (Test de Mann Whitney, (n=9)), le contrôle (les bactéries sur le plastique) est représenté par la colonne la plus à gauche.

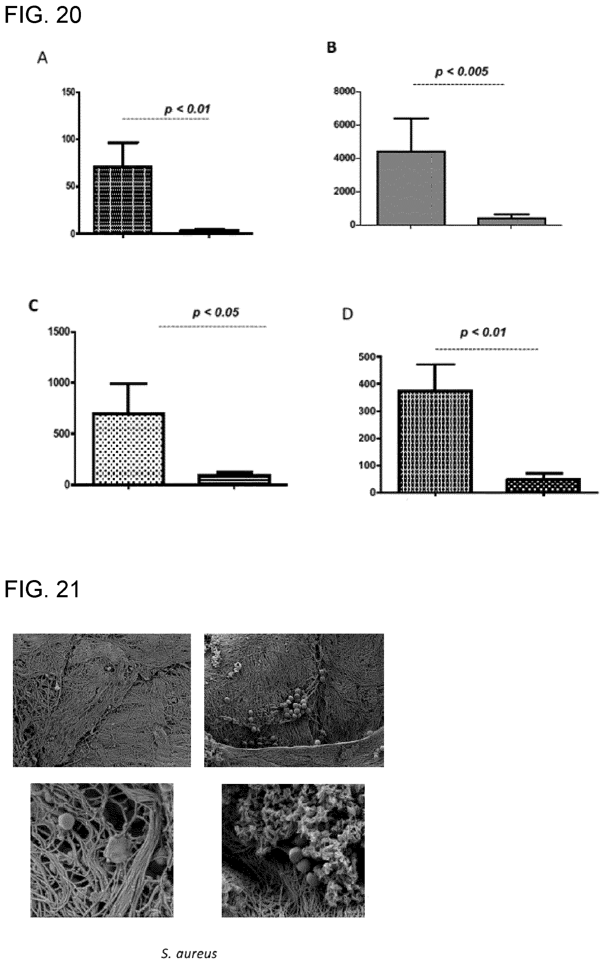

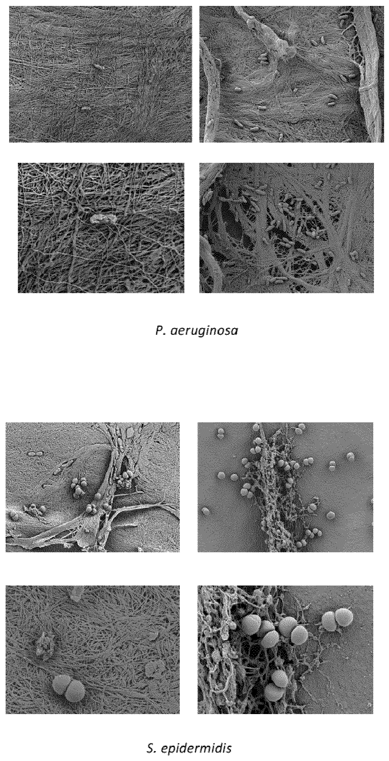

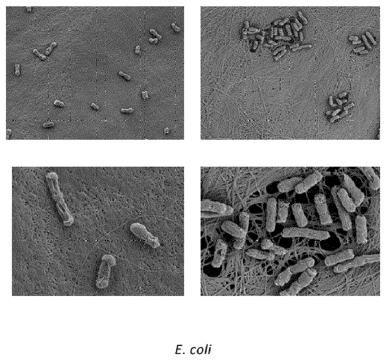

La Fig. 20 représente le pourcentage d'adhésion des bactéries suivantes après 24h d'incubation de*S*. *aureus* (A), *S*. *epidermidis* (B), *E. coli* (C) et *P. aeruginosa* (D) sur les tissus natifs (colonne de gauche) et décellularisés de l'invention (colonne de droite) (Test de Mann Whitney, (n=9)).
La Fig. 21 représente des photographies prises en microscopie électronique à balayage des bactéries adhérées sur tissus natifs (photographies de droite) et décellularisés de l'invention (photographies de gauche).

### [Exemples]

### Préparation de la gelée de Wharton décellularisée :

Les cordons ombilicaux sont récupérés au CHU de Reims puis congelés à -20°C jusqu'à utilisation sans ajout d'antibiotiques ou autre additif. Après rinçage des cordons dans un tampon phosphate salin (PBS, Gibco) pendant 10 mn sous agitation, la gelée de Wharton est récupérée à l'aide de pinces. La veine et les artères sont retirées, puis la gelée est pelée (retrait de la membrane) et découpée en morceaux d'environ 1cm x 1cm sur 1mm d'épaisseur. Ces morceaux sont placés dans des plaques 24 puits et congelés à -20°C avant utilisation.

On rince les morceaux congelés avec 1 mL/puits d'eau distillée pendant 5 min sous agitation (agitateur de plaques, 450 rpm) (température ambiante 20-25°C, l'eau est à température ambiante)

On ajoute 1 mL/puits d'une solution de Triton X-100 diluée au 1/100 dans de l'eau distillée (Triton X-100 (VWR)) et on agite pendant 1h à 37°C

On rince les morceaux de cordon avec de l'eau distillée (1 mL/puits, 2 x 5 mn sous agitation) à température ambiante (20°C à 25°C (bornes incluses))

On ajoute la solution de DNase (DN25 (Sigma) reconstituée dans NaCI 0,15 M (aliquots de 1 mL à 1 mg/mL, dilués au 1/5 dans de l'eau distillée) à 0,2 mg/mL et on agite pendant 24h à 37°C

On rince les morceaux de cordon avec du PBS (1 mL/puits, 2 x 5 mn sous agitation) à température ambiante.

On congèle la plaque à -20°C pendant environ 24h

On congèle jusqu'à -80°C puis on maintient la température à -80°C pendant 24 heures. Le tissu est ensuite mis sous vide à une température de -80°C pendant 24 heures. Il est ainsi lyophilisé.

Pour la préparation du tissu natif, le protocole est identique, mis à part que le Triton et la DNase ne sont pas ajoutés (eau distillée seule à la place).

### A) Caractérisation physicochimique de la matrice extracellulaire décellularisée

### 1) Dosage du matériel génétique du tissu décellularisé

Un premier ensemble d'expériences a été réalisé afin de mettre en évidence la diminution escomptée du matériel génétique du tissu. Pour cela, dans un premier temps, un marquage a été réalisé sur le tissu natif traité comme précédemment indiqué et le tissu décellularisé (tissu sec) selon le procédé de l'invention.

Le matériel génétique a été marqué à l'aide de 4',6-diamidino-2-phénylindole (DAPI, Sigma), puis les tissus ont été observés au microscope à fluorescence.

Les résultats présentés sur la Fig. 1 montrent une absence de noyaux dans le tissu décellularisé, ce qui indiquerait une efficacité du protocole établi. Ces résultats ont été complétés par une coloration histologique à l'hématoxyline/éosine/safran, ainsi que par microspectroscopie infrarouge et une extraction du contenu en acides nucléiques. Les résultats sont visibles sur les Fig. 2 à 4, lesquelles confirment l'absence de noyaux et la diminution de la quantité d'ADN.

Ces différentes approches sont venues confirmer les résultats observés en microscopie à fluorescence : ainsi, sur les coupes histologiques, on constate l'absence de noyaux dans le tissu décellularisé. La microspectroscopie infrarouge (Fig. 3) montre dans le spectre du tissu décellularisé l'absence d'un épaulement correspondant aux acides nucléiques, observé sur le spectre du tissu natif. Enfin, la quantification de l'ADN du tissu sec de l'invention a révélé une quantité d'ADN sous la limite de détection du kit utilisé au laboratoire, soit inférieure à 7,66 ng/mg de tissu sec. (voir Fig. 4).

### 2) Conservation des composants de la matrice extracellulaire

L'efficacité du traitement a été évaluée via l'étude de conservation des composants de la matrice extracellulaire, afin de vérifier que le procédé de l'invention n'altérait pas celle-ci. Des colorations histologiques (non représentées) ont été réalisées, pour visualiser le collagène (l'hématoxyline/éosine/safran) ainsi que les glycosaminoglycanes (Bleu Alcian). Ces coupes ont mis une évidence une absence de dégradation du collagène. Ces résultats, qualitatifs, ont été vérifiés par des méthodes quantitatives.

Ainsi, un dosage du collagène a été réalisé ainsi qu'une quantification des GAGs sulfatés et non sulfatés, composants majeurs du cordon. Les résultats, présentés sur la Fig. 5, montrent une absence de différence entre le tissu natif et le tissu décellularisé, tant dans la quantité de collagène que dans la quantité de GAGs.

La matrice extracellulaire et sa composition ont été également été observées via différentes méthodes de microscopie, présentées dans les Fig. 6 et 7. Ainsi les tissus ont été observés en microscopie optique à lumière polarisée, microscopie électronique à balayage, ainsi qu'en microscopie confocale à balayage laser. Les deux premières techniques n'ont montré aucune différence dans la structure du tissu décellularisé par rapport au tissu natif. La microscopie confocale, via la génération de seconde harmonique, permettant de mettre en évidence les fibres de collagène dans les tissus ainsi que leur orientation, révèle la présence de fibres orientées en condition sèche, perdant cette orientation post-hydratation, pour le tissu décellularisé comme le tissu natif.

Ainsi, l'ensemble de ces résultats montrent que le protocole de décellularisation n'impacte pas la matrice extracellulaire, tant dans sa composition que son organisation.

### 3) Propriétés mécaniques

Les propriétés mécaniques du tissu et leur conservation après décellularisation selon le procédé de l'invention ont également été étudiées.

En référence à la Fig. 8, l'analyse de la porosité au mercure a démontré une distribution similaire des pores pour les deux tissus, avec cependant une augmentation de la taille des pores pour le tissu décellularisé.

Enfin, l'étude du module d'élasticité des tissus a été réalisé par des tests de traction, en conditions sèche et humide. Les tests n'ont montré aucune différence entre le tissu natif et le tissu décellularisé, avec une déformation à 40%, sans rupture du matériau . Le test de traction a été réalisé avec la machine (Universal testing machine, ZWICKY0.5) équipée d'une charge de 10 N. La taille des éprouvettes est de 14x5X1 mm3.

Les résultats relatifs au dosage d'ADN, aux composants de la matrice extracellulaire native et traitée selon le procédé de l'invention ainsi que les propriétés mécaniques sont résumés dans le tableau I suivant :

**Tableau I**

| *Caractéristiques* | Tissu natif | **Tissu décellularisé** |
|---|---|---|
| *ADN (ng*/*µL*/*mg)* | 18,40 | **7,66==>** en dessous du seuil de déctetion du kit |
| *Collagène* (*mg*/*mg)* | 0,90 | 0,89 |
| *Chondroïtine sulfate (µg*/*mg)* | 10,94 | **10,86** |
| *Acide hyaluronique (µg*/*mg)* | 15,11 | **15,09** |
| *Porosité (%)* | 80 | **79** |
| *Module linéaire sec (MPa)* | 13,16 | **14,27** |
| *Module linéaire humide (MPa)* | 2,14 | **1,66** |

### B) Caractérisation des propriétés biologiques

La biocompatibilité du tissu après décellularisation selon le procédé de l'invention ainsi que ses propriétés biologiques intrinsèques ont été étudiées.

### 1) Évaluation de la biocompatibilité

Le tissu décellularisé visant à être proposé sous la dénomination de dispositif médical, celui-ci doit répondre aux normes sur l'évaluation des dispositifs médicaux. Ici nous nous focaliserons sur la norme ISO 10993-5, portant sur les essais de cytotoxicité *in vitro.* Ainsi, la cytotoxicité a été testée sur différents types cellulaires, et au travers de différentes méthodes : la viabilité cellulaire a été évaluée via un test WST-1, visant à quantifier l'activité mitochondriale des cellules ; la toxicité éventuelle a été mise en évidence grâce à la mesure de la lactate déshydrogénase (LDH) dans le milieu de culture ; enfin, une quantification de l'ADN, corrélée au test WST-1, permettrait de distinguer une prolifération cellulaire (activité mitochondriale et quantité d'ADN augmentées) d'un état de stress (activité mitochondriale augmentée mais quantité d'ADN inchangée voire diminuée).

Des cellules stromales (CSMs) de la pulpe dentaire, des CSMs de la gelée de Wharton, des fibroblastes et des ostéoblastes ont été cultivés en monocouche en présence du tissu décellularisé de l'invention. Après 24h de culture, les résultats montrent une viabilité cellulaire supérieure à 70% (seuil limite imposé par la norme), ainsi qu'une absence de libération excessive de la LDH, suggérant la non-cytotoxicité du tissu décellularisé (voir Fig. 10).

Les CSMs de la gelée de Wharton ont ensuite été ensemencées sur les tissus décellularisés afin d'évaluer leur potentielle adhésion. Après 7 jours de culture de ces cellules sur les tissus décellularisés, une quantification de l'ADN révèle la présence de cellules adhérées sur les tissus (voir Fig. 11). Ces résultats ont été confirmés par une observation des CSMs marquées et adhérées sur le tissu par microscopie confocale, ainsi que par une coloration histologique, où l'on observe une colonisation du tissu par les CSMs.

### 2) Évaluation de la bioactivité

La bioactivité des tissus a été évaluée par une analyse en spectrométrie de masse, ainsi que par des tests ELISA. La première méthode vise à mettre en évidence la libération de peptides, la seconde celle de facteurs de croissance.

Après 72h d'incubation des tissus dans du milieu biologique, les peptides libérés dans le milieu ont été identifiés par analyse protéomique. Le tissu natif libère principalement des peptides issus du métabolisme cellulaire. En revanche, le tissu décellularisé ne libère que 60% de ces mêmes peptides, mais également 35% de peptides liés à la matrice extracellulaire, notamment la régulation de la réponse immunitaire, la cicatrisation tissulaire (7 peptides à savoir : Tenascin; Keratin, type II cytoskeletal 6A; Collagen alpha-1(III) chain; Fibronectin; Keratin, type II cytoskeletal 1; Keratin, type **I** cytoskeletal 17; Keratin, type **I** cytoskeletal 9), et à une activité antimicrobienne (3 peptides à savoir : Fibrinogen beta chain, Fibrinogen alpha chain, Keratin, type **I** cytoskeletal 10).

Une quantification par le test ELISA de trois facteurs favorisant la réparation tissulaire, à savoir le VEGF, l'HGF et le TGF-β, a montré qu'après 72h d'incubation dans un milieu biologique, le tissu décellularisé libérait significativement plus de VEGF et d'HGF (Fig. 12). En référence à la Fig. 12, on constate que la gelée de l'invention libère plus de VEGF et plus d'HGF que le tissu natif, ce qui suggère une libération facilitée de ces derniers du fait probablement de l'augmentation de la taille des pores dans le cas de la matrice de l'invention, bien que les Demandeurs ne soient pas liés à cette explication..

### 3) Évaluation des propriétés anti-inflammatoires

### Effet sur la sécrétion de cytokines pro inflammatoire par les monocytes circulants

L'un des points clés en médecine régénératrice est de favoriser une cicatrisation sans engager d'inflammation. Les propriétés anti-inflammatoires des tissus ont donc été testées. Le tissu décellularisé a ainsi été mis au contact de monocytes circulants, pendant 4h et 24h. La sécrétion de cytokines pro-inflammatoires, l'IL-1β et le TNF-α, a été évaluée grâce à un test ELISA (Fig. 13). Il a été observé qu'au contact des tissus, les monocytes étaient activés, à un niveau compris entre le contrôle négatif (culture sur matériau inerte ; verre) et le contrôle positif (culture en présence d'un stimulus inflammatoire ; lipopolysaccharide). Cette expérience met donc en évidence une stimulation modérée des cellules du système immunitaire par le tissu décellularisé. Ces niveaux de sécrétion ont été rapportés à ceux d'une culture de monocytes en présence du stimulus inflammatoire (LPS : lipopolysaccharide) (Fig. 14). On observe ainsi que la présence d'un stimulus inflammatoire n'entraîne pas d'augmentation de la sécrétion des cytokines pro-inflammatoires par les monocytes cultivés en présence du tissu, confirmant son effet anti-inflammatoire.

### Effet sur l'action Phagocytaires des PNNs

Une seconde série d'expériences a été menée sur un autre type cellulaire du système immunitaire, les polynucléaires neutrophiles humains (PNN). Ces cellules sont les premières à arriver au niveau du site post-implantation. Leur activation exacerbée peut endommager le processus de réparation tissulaire. Les propriétés phagocytaires des PNN en présence du tissu décellularisé ont été étudiées par cytométrie en flux.

La phagocytose de particules de tailles similaires à des bactéries (pHRodo^{®}), en présence de surnageants obtenus du tissu natif et du tissu décellularisé après 72h d'incubation en milieu biologique à 37°C, n'a pas été bloquée (pour le contrôle le pourcentage de particules phagocytées est de 45,56%, 43,31% pour le tissu natif et 40,39% pour la gelée de l'invention (voir Fig. 15). Ces résultats suggèrent que les tissus n'impactent pas l'action phagocytaire des neutrophiles.

### Effet sur la production de ROS

De la même façon, le mécanisme d'élimination des antigènes impliquant la production d'espèces réactives de l'oxygène (ROS) et leur quantification a été étudiée (Fig. 16). L'intensité médiane de fluorescence est de 6000 pour le tissu natif et de 5000 pour le tissu décellularisé de l'invention. Le ratio LPS (stimulus inflammatoire)/basal avoisine 1 pour le tissu natif et le tissu de l'invention. Ainsi, la présence du tissu décellularisé n'a pas induit de modification significative de la production de ROS, par rapport à la présence de tissu natif. La même expérience a été conduite en stimulant les PNN avec du LPS, et aucun effet cumulatif n'a été observé.

### Effet sur la sécrétion d'IL-8 par les PNNs

Enfin, une quantification par ELISA de la sécrétion d'IL-8 par les PNN en présence des tissus a été réalisée (Fig. 17A et B) : de la même façon que les ROS, la quantité d'IL-8 produite par les PNN n'est significativement pas plus importante en présence du tissu décellularisé, par rapport au tissu natif, et aucun effet cumulatif n'a été observé. On observe dans le deux cas une production de 90 pg/mL d'IL-8 sans stimulus et un ratio de production d'IL-8 LPS/basal est d'environ 1,5 pour les deux tissus. En résumé, les propriétés anti-inflammatoires des tissus n'impactent pas le bon fonctionnement des PNN.

### Effet sur la migration des fibroblastes

Enfin, des expériences de migration de fibroblastes ont été menées, afin de mettre en évidence un effet anti-fibrotique. En effet, une trop grande population de fibroblastes sur le site d'une lésion ou sur un matériau visant à combler cette lésion peut conduire à une hyper-cicatrisation : les fibroblastes, en grand nombre, sécrétant une grande quantité de matrice extracellulaire, altèrent le tissu ou l'organe touché, et donc son bon fonctionnement. Les résultats observés sont présentés sur la Fig. 18. On observe qu'en présence du tissu décellularisé, les fibroblastes ne migrent pas ou très peu, suggérant une propriété anti-fibrotique de la membrane après traitement de décellularisation.

### 4) Évaluation des propriétés antimicrobiennes

L'analyse protéomique ayant montré la présence de peptides antimicrobiens dans le tissu décellularisé, son effet sur la croissance et l'adhésion des bactéries a été testé. Les expériences ont été menées sur 4 bactéries : deux Gram+, *Staphylococcus aureus* et *Staphylococcus epidermidis* ; deux Gram-, *Pseudomonas aeruginosa* et *Escherichia coli.*

Suite à la mise en culture de bactéries en présence du tissu décellularisé, la croissance planctonique a été évaluée par mesure de la densité optique (Fig. 19). On observe, quelle que soit la bactérie considérée, un ralentissement de leur croissance.

Les différentes bactéries adhérées sur les tissus ont été détachées par ultrasons puis dénombrées (Fig. 20). De la même façon que pour la croissance planctonique, on observe une diminution significative du nombre de bactéries adhérées sur le tissu décellularisé. En complément du dénombrement, des prises de vue en microscopie confocale et en microscopie électronique à balayage ont été réalisées et confirment les résultats observés précédemment (Fig. 21): une faible quantité de bactéries adhérées sur les tissus décellularisé, et une altération de ces bactéries.

Le mécanisme privilégié pour expliquer ces résultats est un phénomène de « contact killing ».

La gelée de Wharton décellularisée de l'invention répond aux exigences des normes des dispositifs médicaux, et ne présente aucune cytotoxicité vis à vis des cellules humaines, ni de profil inflammatoire. Le procédé de décellularisation n'impacte pas la composition ni les propriétés biomécaniques du tissu. Les propriétés intrinsèques de la gelée décellularisée à savoir, antibactériens et bioactifs présentent un intérêt majeur dans la cicatrisation tissulaire.

## Revendications

1. Gelée de Wharton décellularisée, **caractérisée en ce qu'**elle contient moins de 50 ng d'ADN double brin/mg de tissu sec et,
- au moins 5 µg de glycosaminoglycanes sulfatés /mg de tissu sec, en particulier au moins 7 µg de glycosaminoglycanes sulfatés /mg de tissu sec et plus particulièrement au moins 10 µg de glycosaminoglycanes sulfatés /mg de tissu sec et/ou
- au moins 8 µg de glycosaminoglycanes non sulfatés /mg de tissu sec, en particulier au moins 14 µg de glycosaminoglycanes non sulfatés /mg de tissu sec.

2. Gelée selon la revendication 1, **caractérisée en ce qu'**elle contient
- au moins 5 µg chondroïtine sulfate /mg de tissu sec, en particulier au moins 7 µg de chondroïtine sulfate /mg de tissu sec et plus particulièrement au moins 10 µg de chondroïtine sulfate /mg de tissu sec et/ou
- au moins 8 µg d'acide hyaluronique /mg de tissu sec, en particulier au moins 14 µg d'acide hyaluronique /mg de tissu sec.

3. Gelée de Wharton décellularisée selon la revendication 1 ou 2, **caractérisée en ce qu'**elle présente une porosité supérieure à 60%, en particulier supérieure ou égale à 70% et plus particulièrement égale ou supérieure à 80% et inférieure ou égale à 85%.

4. Gelée de Wharton selon l'une quelconque des revendications 1 à 3, **caractérisée en ce qu'**elle contient en outre, au moins 0,70 mg de collagène /mg de tissu sec et en particulier au moins 0,80 mg de collagène /mg de tissu sec.

5. Gelée de Wharton selon l'une quelconque des revendications précédentes, **caractérisée en ce qu'**elle est lyophilisée ou obtenue par réhydratation de la gelée lyophilisée.

6. Gelée de Wharton selon l'une quelconque des revendication précédentes, **caractérisée en ce qu'**elle se présente sous une forme divisée, en particulier sous forme de poudre.

7. Gelée de Wharton selon l'une quelconque des revendications précédentes **caractérisée en ce qu'**elle provient d'un cordon ombilical et notamment d'un cordon ombilical humain.

8. Gelée de Wharton selon l'une quelconque des revendications précédentes, **caractérisée en ce qu'**elle contient au moins un peptide antimicrobien et/ou au moins un facteur de croissance et/ou au moins un peptide favorisant la cicatrisation tissulaire.

9. Gelée de Wharton selon la revendication 8, **caractérisée en ce que** ledit peptide anti-microbien est choisi parmi le fibrinogène chaîne beta, le fibrinogène chaine alpha et la cytokératine -10, **en ce que** ledit facteur de croissance est choisi parmi VEGF, HGF et TGF-beta et **en ce que** ledit peptide favorisant la cicatrisation est choisi parmi la ténascine, la cytokératine-6A, le collagène alpha-1 (III), la fibronectine, la cytokératine -1, la cytokératone-17 et la cytokératine-9.

10. Gelée de Wharton selon l'une quelconque des revendications précédentes, **caractérisée en ce que** l'augmentation de la sécrétion des cytokines pro inflammatoires par les monocytes circulants en présence de ladite gelée est inférieure à celle due à un facteur inflammatoire, en particulier un lipopolysaccharide (LPS) et/ou **en ce qu'**en présence de ladite gelée le nombre de cellules phagocytées par les PNNs est égal au moins 70% et en particulier 80% ou plus du nombre de cellules ayant phagocyté les particules en l'absence de ladite gelée et/ou **en ce que** la quantité de ROS secrétée par les PNNs en présence de ladite gelée est inférieure ou égale à la quantité de ROS secrété par les PNNs en présence d'un stimulus inflammatoire, en particulier un lipopolysaccharide et/ou **en ce que** le ratio [production d'IL-8 par les PNN en présence d'un stimulus inflammatoire, en particulier un polysaccharide LPS en présence de ladite gelée]/[la production d'IL-8 par les PNNs en présence de cellules basales] est inférieur ou égal à 1,5 et/ou **en ce que** le pourcentage de fibroblastes ayant migré pendant 48h en présence de ladite gelée est inférieur au pourcentage de fibroblastes ayant migré pendant 48h en l'absence de ladite gelée.

11. Biomatériau **caractérisé en ce qu'**il comporte ou est constitué de la gelée de Wharton selon l'une quelconque des revendications 1 à 10.

12. Composition cosmétique ou pharmaceutique **caractérisée en ce qu'**elle comprend en tant qu'ingrédient actif, la gelée de Wharton selon l'une quelconque des revendications 1 à 10.

13. Gelée selon l'une quelconque des revendications 1 à 10 ou composition pharmaceutique selon la revendication 12, pour son utilisation en tant que médicament et en particulier en qu'agent favorisant la cicatrisation, agent réduisant l'inflammation et/ou agent hydratant pour le traitement d'une maladie ou dans une condition d'écart à la norme non pathologique nécessitant un tel traitement.

14. Produit choisi parmi un implant, un pansement, un greffon, une préparation pour application topique, une bio encre, un agent d'enrobage cellulaire **caractérisé en ce qu'**il contient ou est constitué une gelée selon l'une quelconque des revendications 1 à 10.

15. Procédé de décellularisation d'un tissu conjonctif œdémateux, en particulier la gelée de Wharton selon lequel :
- on traite ledit tissu de préférence préalablement congelé à -20°C avec une solution de tensioactif anionique en maintenant la température à 37°C ;
- après rinçage à l'eau distillée à température ambiante, on ajoute une DNase et l'on maintient à 37°C ;
- on rince ensuite avec du PBS et éventuellement on lyophilise le tissu traité.
